(19) Europäisches Patentamt
European Patent Office
Office européen des brevets

(11) Veröffentlichungsnummer: **0 635 493 A2**

# (12) EUROPÄISCHE PATENTANMELDUNG

(21) Anmeldenummer: **94103735.0**

(22) Anmeldetag: **11.03.94**

(51) Int. Cl.[6]: **C07D 217/26**, C07D 263/20, C07D 413/06, C07D 209/48, C07C 229/34

(30) Priorität: **24.03.93 CH 896/93**

(43) Veröffentlichungstag der Anmeldung: **25.01.95 Patentblatt 95/04**

(84) Benannte Vertragsstaaten: **AT BE CH DE DK ES FR GB IT LI NL**

(71) Anmelder: **F. HOFFMANN-LA ROCHE AG**
**Postfach 3255**
**CH-4002 Basel (CH)**

(72) Erfinder: **Hilpert, Hans**
**12 Fasanenstrasse**
**CH-4153 Reinach (CH)**

(74) Vertreter: **Kellenberger, Marcus, Dr. et al**
**Grenzacherstrasse 124**
**Postfach 3255**
**CH-4002 Basel (CH)**

(54) **Verfahren zur Herstellung eines beta-Aminoalkohols.**

(57) Verfahren zur Herstellung des 2-[3(S)-Amino-2-(R)-hydroxy-4-phenylbutyl]-N-tert.butyl-decahydro-(4aS,8aS)-isochinolin-3(S)-carboxamids der Formel

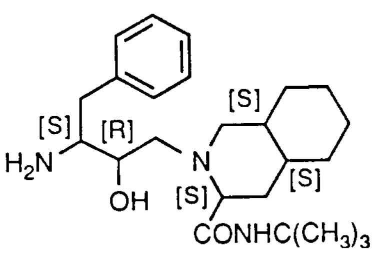

10

via dem Aminoalkohol 10 entsprechendem Oxazolidinon, sowie in diesem Verfahren vorkommende Zwischenprodukte.

EP 0 635 493 A2

Die vorliegende Erfindung betrifft ein neues Verfahren zur Herstellung eines β-Aminoalkohols, nämlich des 2-[3(S)-Amino-2(R)-hydroxy-4-phenylbutyl]-N-tert.butyl-decahydro-(4aS,8aS)-isochinolin-3(S)-carboxamids der Formel

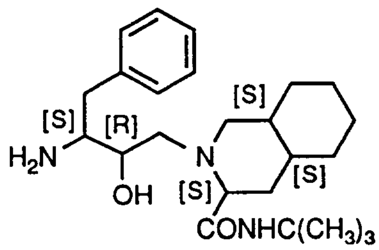

10

Ferner betrifft die Erfindung neue im besagten Verfahren vorkommende Zwischenprodukte.

Das erfindungsgemässe Verfahren ist dadurch gekennzeichnet, dass man

1.a) einen Sulfonsäureester der Formel

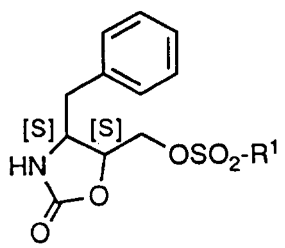

VII

worin $R^1$ nieder-Alkyl oder gegebenenfalls durch bis zu 2 Halogenatome oder nieder-Alkyl- oder Nitrogruppen substituiertes Phenyl ist, in Gegenwart von einer Base mit dem N-tert.Butyl-decahydro-(4aS,8aS)-isochinolin-3(S)-carboxamid der Formel

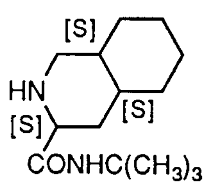

8

umsetzt, das entstandene Oxazolidinon der Formel

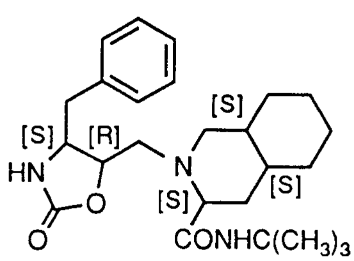

9

mit einer starken Säure in ein Salz des Oxazolidinons der Formel 9 überführt, dieses Salz mit einer Base versetzt und

1.b) das erhaltene Oxazolidinon der Formel 9 mit einer Base zum Aminoalkohol der obigen Formel 10 aufspaltet.

Die obigen Verbindungen 8 und 10 entsprechen denjenigen der Formeln VII bzw. II in der EPA-0432695, wo sie als Zwischenprodukte in der Herstellung von therapeutisch wirksamen Produkten beschrieben sind.

Die Reaktion eines Sulfonsäureesters der Formel VII mit dem Amid 8 wird zweckmässigerweise in einem Lösungsmittel, wie DMSO, einem Kohlenwasserstoff, z.B. Toluol, Triäthylamin oder einem nieder-Alkanol, z.B. Aethanol, oder einem Keton, vorzugsweise in 4-Methyl-2-pentanon, und in Gegenwart einer Base, wie einem nieder-Alkylamin oder einem Alkalimetallcarbonat, vorzugsweise Triäthylamin oder Natriumcarbonat, unter Erhitzen bis zur Rückflusstemperatur, vorzugsweise bei 50-150 °C, insbesondere bei 80-110 °C durchgeführt. Zur Reinigung des entstandenen Oxazolidinons 9 wird ein leicht zu kristallisierendes Salz, wie ein Sulfonat, inbesondere das p-Tolyl-, p-Bromphenyl- oder p-Nitrophenylsulfonat, durch Zugabe einer starken Säure, wie Schwefel- oder vorzugsweise Salzsäure, hergestellt.

Die Aufspaltung des Oxazolidinons 9 nach vorgängiger extraktiven Entfernung der Sulfonsäure mit einer Base, vorzugsweise Natriumbicarbonat, in einem Lösungsmittel, vorzugsweise Aethylacetat, wird zweckmässigerweise in einem Lösungsmittel, wie Wasser, Aethanol oder einem Gemisch davon, mittels einer Base wie NaOH oder KOH, unter Erhitzen bis zur Rückflusstemperatur, vorzugsweise bei 20-100 °C, insbesondere bei 80 °C, bewerkstelligt.

Die obigen Sulfonsäureester VII werden erfindungsgemäss dadurch hergestellt, dass man

2.a) einen α-Hydroxy-β-aminosäureester der Formel

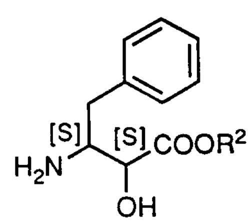

IV

worin $R^2$ nieder-Alkyl ist, oder ein Salz davon mittels eines Carbonylierungsmittels cyclisiert,

2.b) im so erhaltenen 2-Oxo-oxazolidin-5-carbonsäureester der Formel

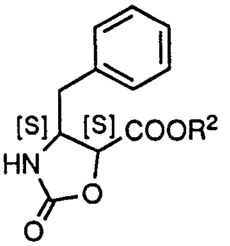

V

die Carbo-niederalkoxygruppe zur Hydroxymethylgruppe reduziert und

2.c) den erhaltenen (4S,5S)-4-Benzyl-5-hydroxymethyl-oxazolidin-2-on der Formel

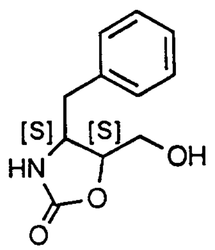

6

in Gegenwart einer Base mit einem Sulfonsäurechlorid $R^1$-$SO_2Cl$ in einen Sulfonsäureester VII umwandelt.

Im Rahmen der vorliegenden Erfindung bezeichnet der Ausdruck nieder-Alkyl allein (z.B. für $R^1$) oder in Kombination (z.B. für nieder-Alkanol) geradkettige oder verzweigte Kohlenwasserstoffreste mit bis zu 6, vorzugsweise 4 C-Atome. Beispiele davon sind Methyl, Aethyl und Isopropyl. Beispiele von Salzen des α-Hydroxy-β-aminosäureesters IV sind solche mit einer Säure, wie Essigsäure oder Schwefelsäure.

Beispiele von Carbonylierungsmittel sind Carbonyldiimidazol, Chlorameisensäurephenylester, Triphosgen und vorzugsweise Phosgen. Die Carbonylierung von IV oder eines Salzes davon wird zweckmässigerweise in einem Lösungsmittel, wie $CH_2Cl_2$, Toluol oder vorzugsweise THF, bei einer Temperatur zwischen -10 und +50 °C, vorzugsweise zwischen 0 und 20 °C, gegebenenfalls in Gegenwart einer Base, wie Triäthylamin, $K_2CO_3$ oder $NaHCO_3$, durchgeführt.

Die Reduktion des 2-Oxo-oxazolidin-5-carbonsäureesters V wird zweckmässigerweise in einem Lösungsmittel, wie Toluol, THF oder vorzugsweise Aethanol, bei einer Temperatur zwischen 0 bis 60 °C, insbesondere bei 22 °C, mittels Natrium-bis-(2-methoxyäthoxy)aluminiumhydrid, $LiAlH_4$ oder vorzugsweise $NaBH_4$ bewerkstelligt.

Die Sulfonierung des Alkohols 6 wird in einem Lösungsmittel, wie Aethylacetat, Aceton oder vorzugsweise THF, in Gegenwart einer Base, wie Triäthylamin oder vorzugsweise N-Methylmorpholin, bei einer Temperatur zwischen 0 und 60 °C, insbesondere zwischen 20 und 40 °C durchgeführt.

Die obigen α-Hydroxy-β-aminosäureester IV lassen sich erfindungsgemäss dadurch herstellen, dass man

3.a) das 3-Phenyl-2(S)-phthalimidopropan-1-al der Formel

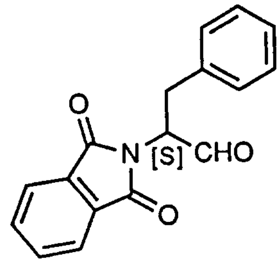

1

in das 1-Cyano-3-phenyl-2(S)-phthalimidopropan-1-olderivat der Formel

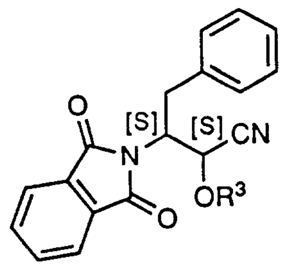

II

worin $R^3$ H, nieder-Alkanoyl, Benzyloxycarbonyl oder Triniederalkylsilyl ist,
umwandelt,

3.b) eine im Nitril der Formel II enthaltene Triniederalkylsilylgruppe abspaltet,

3.c) das erhaltene Nitril der Formel II, worin $R^3$ H, nieder-Alkanoyl oder Benzyloxycarbonyl ist, in Gegenwart eines nieder-Alkanols $R^2$-OH mit einer starken Säure in ein Salz des Iminoäthers der Formel

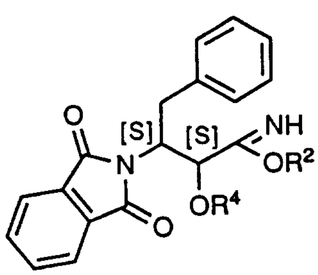

II'

worin $R^4$ H, nieder-Alkanoyl oder Benzyloxycarbonyl und $R^2$ nieder-Alkyl ist,
umwandelt,

3.d) das Salz des Iminoäthers der Formel II' gegebenenfalls isoliert, dann hydrolysiert und

3.e) den erhaltenen α-Hydroxysäureester der Formel

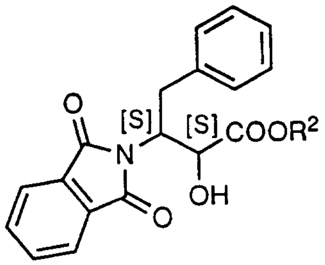

III

zunächst mit einer Base und dann einer starken Säure in den α-Hydroxy-β-aminosäureester der Formel IV umwandelt.

Zur Herstellung eines Nitrils II, worin $R^3$ Wasserstoff ist, wird eine Lösung des Aldehyds 1, z.B. in Toluol, mit wässrigem Natriumpyrosulfit versetzt und das entstandene Additionsprodukt von Pyrosulfit und dem Aldehyd 1 wird gegebenenfalls in einem Lösungsmittel, wie Wasser oder gegebenenfalls wässriges Dichlormethan bzw. Toluol, mit NaCN behandelt. In einer Variante wird ein Gemisch des Aldehyds 1 und von $ZnBr_2$ in einem Lösungsmittel, wie $CH_2Cl_2$, bei -70 bis 0 ° C, z.B. -15 ° C, mit Trimethylsilylcyanid umgesetzt und der entstandene Silyläther des Cyanhydrins durch Zugabe einer Lösung von Zitronensäure in Aethanol gespalten.

Zur Herstellung eines Nitrils II, worin $R^3$ nieder-Alkanoyl, z.B. Acetyl ist, wird eine Lösung des Aldehyds 1 und des entsprechenden Säureanhydrids, wie Acetanhydrid, in $CH_2Cl_2$ in Gegenwart von Benzyltriäthylammoniumchlorid mit NaCN unter Abkühlen, zweckmässig auf -10 bis 0 ° C, versetzt. Zur Herstellung eines Nitrils II, worin $R^3$ Benzyloxycarbonyl ist, wird analog vorgegangen, jedoch unter Verwendung von Chlorameisensäurebenzylester an Stelle eines Säureanhydrids.

Durch Hydrierung des Nitrils II, worin $R^3$ Benzyloxycarbonyl ist, in einem Lösungsmittel, wie Aethanol oder $CH_2Cl_2$, erhält man das Nitril II, worin $R^3$ Wasserstoff ist.

Durch Umsetzung des Cyanhydrins II in Gegenwart eines nieder-Alkanols $R^2$-OH mit einer starken Säure, wie HCl, in einem Lösungsmittel, wie einem nieder-Alkanol $R^2$-OH, $CH_2Cl_2$, Toluol, t-Butylmethyläther oder vorzugsweise einem Gemisch von $CH_2Cl_2$ und $R^2$-OH oder von Toluol und $R^2$-OH, unter Abkühlen auf -10 bis + 10 ° C zweckmässig auf 0 ° C, erhält man das entsprechende Salz des Iminoäthers der Formel II'.

Dieses wird z.B. mit wässrigem Aethylacetat, mit $CH_2Cl_2$ oder vorzugsweise mit wässrigem Toluol und $R^2$-OH zum Hydroxysäureester III hydrolysiert.

Die Trennung der Isomeren erfolgt durch Kristallisation eines Salzes, z.B. des Hydrochlorids, des (S,S)-Iminoäthers der Formel II' oder des α-Hydroxysäureesters III und gibt eine Isomerenreinheit von 93-99%, falls man $CH_2Cl_2$ als Lösungsmittel verwendet. Bei Verwendung von Toluol als Lösungsmittel ist die Isomerentrennung des Salzes von II' nicht möglich. Eine Anreicherung bis zu 92% bzw. 99% am erwünschten Isomeren wird erst durch Kristallisation der Verbindung IV bzw. 6 erreicht. Alle Verbindungen III können auch durch Chromatographie an $SiO_2$ getrennt werden.

Durch Behandlung einer Verbindung III zunächst mit einer Base, wie Methylamin, und dann mit einer starken Säure, wie HCl, in einem Lösungsmittel, wie THF oder vorzugsweise einem Alkohol, wie $R^2$-OH, insbesondere Methanol, bei einer Temperatur von -10 bis + 20 ° C, vorzugsweise bei 0 ° C für die 1. Stufe und bei 20 ° C für die 2. Stufe, erhält man die Verbindung der Formel IV.

Den Ausgangsaldehyd 1 erhält man durch Erhitzen von L-Phenylalanin mit Phthalsäureanhydrid in Toluol, Umsetzung des entstandenen N-geschützten L-Phenylalanins mit Oxalylchlorid in Toluol und katalytischen Mengen DMF, gefolgt durch katalytische (Pd/C) Hydrierung des entstandenen, dem erwünschten Aldehyd 1 entsprechenden Säurechlorids in Gegenwart von 1,2-Butylenoxid in Toluol.

<u>Beispiel 1</u> (VII + 8→9, $R^1$ = p-$NO_2C_6H_4$)

A) Eine Suspension von 24,69 g p-Nitrobenzolsulfonsäure-(4S,5S)-4-benzyl-2-oxo-oxazolidin-5-ylmethylester (Beispiel 12), 15,0 g N-tert.Butyl-decahydro-(4aS,8aS)-isochinolin-3(S)-carboxamid und 10,0 g Natriumcarbonat in 76 ml 4-Methyl-2-pentanon wird unter Rühren 10 Stunden unter Rückfluss erhitzt. Die Suspension wird auf 80 ° C abgekühlt, mit 176 ml 4-Methyl-2-pentanon und 54 ml 3N Salzsäure verdünnt, auf 40 ° C abgekühlt und filtriert. Der Rückstand wird mit Wasser und 4-Methyl-2-pentanon gewaschen und zu 35,1 g (88%) (3S,4aS,8aS)-2-[-(4S,5R)-4-Benzyl-2-oxo-oxazolidin-5-ylmethyl]-N-tert.butyl-decahydro-isochinolin-3-carboxamid-p-nitrobenzolsulfonat getrocknet, $[\alpha]_D^{20}$ = -31,2 ° (1% in DMF).

B) Man erhält das gleiche Salz in 89% Ausbeute bei Verwendung von Triethylamin (2 Aequivalente) anstelle von Natriumcarbonat.

<u>Beispiel 2</u> (VII + 8→9, $R^1$ = $CH_3$)

Analog Beispiel 1B) erhält man durch Umsetzung von 14 g Methansulfonsäure-(4S,5S)-4-benzyl-2-oxo-oxazolidin-5-ylmethylester (Beispiel 9) mit 11,7 g N-tert.Butyl-decahydro-(4aS,8aS)-isochinolin-3(S)-carboxamid nach Zugabe von 1 Aequivalent p-Tolylsulfonsäure 11,8 g (40%) (3S,4aS,8aS)-2-[(4S,5R)-4-Benzyl-2-oxo-oxazolidin-5-ylmethyl]-N-tert.butyl-decahydroisochinolin-3-carboxamid-p-tolylsulfonat, Smp. 203-205 °C.

Beispiel 3 (VII + 8→9, $R^1$ = p-Tolyl)

Analog Beispiel 1B) erhält man durch Umsetzung von 7,2 g p-Toluolsulfonsäure-(4S,5S)-4-benzyl-2-oxo-oxazolidin-5-ylmethylester (Beispiel 11) mit 4,8 g N-tert.Butyl-decahydro-(4aS,8aS)-isochinolin-3(S)-carboxamid 5,4 g (45%) (3S,4aS,8aS)-2-[(4S,5R)-4-Benzyl-2-oxo-oxazolidin-5-ylmethyl]-N-tert.-butyl-decahydro-isochinolin-3-carboxamid-p-tolylsulfonat, Smp. 202-204 °C.

Beispiel 4 (VII + 8→9, $R^1$ = p-$BrC_6H_4$)

Analog Beispiel 1B) erhält man durch Umsetzung von 30 g p-Brombenzolsulfonsäure-(4S,5S)-4-benzyl-2-oxo-oxazolidin-5-ylmethylester (Beispiel 13) mit 16,8 g N-tert.Butyl-decahydro-(4aS,8aS)-isochinolin-3(S)-carboxamid 36,5 g (78%) (3S,4aS,8aS)-2-[(4S,5R)-4-Benzyl-2-oxo-oxazolidin-5-ylmethyl]-N-tert.butyl-decahydro-isochinolin-3-carboxamid-p-bromphenylsulfonat, $[\alpha]_D^{20}$ : -29,9 ° (1% in DMF).

Beispiel 5 (VII + 8→9, $R^1$ = o-$NO_2C_6H_4$)

Analog Beispiel 1B) erhält man durch Umsetzung von 39,2 g o-Nitrobenzolsulfonsäure-(4S,5S)-4-benzyl-2-oxo-oxazolidin-5-ylmethylester (Beispiel 10) mit 23,8 g N-tert.Butyl-decahydro-(4aS,8aS)-isochinolin-3(S)-carboxamid nach Zugabe von 1 Aequivalent p-Tolylsulfonsäure 42 g (70%) (3S,4aS,8aS)-2-[(4S,5R)-4-Benzyl-2-oxo-oxazolidin-5-ylmethyl]-N-tert.butyldecahydro-isochinolin-3-carboxamid-p-tolylsulfonat, $[\alpha]_D^{20}$ : -34,2 ° (1% in DMF).

Beispiel 6 (9→10)

34,7 g des Salzprodukts von Beispiel 1 werden zwischen 110 ml Aethylacetat und 110 ml gesättigtem Natriumbicarbonat verteilt. Die wässrige Schicht wird mit Aethylacetat extrahiert und die Aethylacetat-Extrakte mit 110 ml gesättigtem Natriumbicarbonat und mit Wasser gewaschen. Die organischen Extrakte werden eingedampft, der Rückstand mit 55 ml Aethanol verdünnt und mit einer Lösung von 11,0 g Natriumhydroxid in 55 ml Wasser unter Rühren versetzt. Das Gemisch wird unter Rückfluss 5 Stunden erhitzt, mit 55 ml $H_2O$ verdünnt und auf 22 °C abgekühlt. Die Suspension wird filtriert und der Rückstand bis zu neutralem pH des Filtrats mit Wasser gewaschen. Der Rückstand wird zu 20,7 g (93%) 2-[3(S)-Amino-2(R)-hydroxy-4-phenylbutyl]-N-tert.butyl-decahydro-(4aS,8aS)-isochinolin-3(S)-carboxamid getrocknet, Smp. 175-176 °C.

Beispiel 7 (IV→V)

A) Einer Suspension von 43 g eines 92:8-Gemisches der (2S,3S)- und (2R,3S)-Isomeren des 3-Amino-2-hydroxy-4-phenyl-buttersäuremethylesteracetats (Beispiel 23B) in 350 ml THF werden bei 0 °C 39 g Phosgen eingeleitet. Nach 1 Stunde Rühren bei 0 °C und 24 Stunden bei 22 °C wird die Lösung zur Trokkene eingeengt, der Rückstand in 200 ml $CH_2Cl_2$ gelöst und mit Wasser und gesättigten $NaHCO_3$ gewaschen. Die Wasserphasen werden mit $CH_2Cl_2$ extrahiert und die vereinigten $CH_2Cl_2$-Phasen getrocknet und filtriert. Das Filtrat wird zu einem rohen 92:8-Gemisch der (4S,5S)- und (4S,5R)-Isomeren des 4-Benzyl-2-oxo-oxazolidin-5-carbonsäuremethylesters eingedampft. DC ($SiO_2$, Aethylacetat): $R_f$ = 0,5. Ausbeute 100%.

B) Analog Beispiel 7A) erhält man durch Umsetzung eines 98:2-Gemisches der (2S,3S)- und (2R,3S)-Isomeren des 3-Amino-2-hydroxy-4-phenyl-buttersäuremethylester-acetats (Beispiel 23A) ein rohes 98:2-Gemisch der (4S,5S)- und (4S,5R)-Isomeren des 4-Benzyl-2-oxo-oxazolidin-5-carbonsäuremethylesters in 100% Ausbeute. DC ($SiO_2$, Aethylacetat): $R_f$ = 0,5.

Beispiel 8 (V→6)

A) Zu einer gerührten Lösung von 20,8 g $NaBH_4$ in 360 ml Aethanol wird bei 15-20 °C während 1,5 Stunden 150 g des 98:2-Gemisches der (4S,5S)- und (4S,5R)-Isomeren des 4-Benzyl-2-oxo-oxazolidin-5-carbonsäuremethylesters von Beispiel 7B) gegeben und 2 Stunden nachgerührt. Die Suspension wird bei 20 °C mit 540 ml Wasser versetzt und der pH mit 165 ml 3N Salzsäure auf 7 gebracht. Die Suspension wird 2,5 Stunden bei 22 °C gerührt, 18 Stunden bei 4 °C stehen gelassen, dann abfiltriert und der Rückstand mit Wasser gewaschen und zu 111,6 g (84%) 99%-igem (4S,5S)-4-Benzyl-5-hydroxymethyloxazolidin-2-on, Smp. 167,3-168,9 °C, $[\alpha]_D^{20}$ : -79,4 ° (1% in Methanol) getrocknet.

B) Analog Beispiel 8A) erhält man aus dem 92:8-Gemisch der (4S,5S)- und (4S,5R)-Isomeren von Beispiel 7A) in 75% Ausbeute 99%iges (4S,5S)-4-Benzyl-5-hydroxymethyl-oxazolidin-2-on, Smp. 167-169 °C.

Beispiel 9 (6→VII, $R^1$ = $CH_3$)

Zu einer Suspension von 10,4 g des Alkohols von Beispiel 8 in 20 ml Aceton und 6,1 ml N-Methylmorpholin wird bei 25°C eine Lösung von 4,7 ml Methansulfochlorid in 10 ml Aceton gegeben und die Suspension 3 Stunden bei 25°C gerührt. Es werden erneut 1,1 ml N-Methylmorpholin zugegeben und es wird 1 Stunde gerührt. Die Suspension wird mit 80 ml halbgesättigter $NaHCO_3$-Lösung und Aethylacetat geschüttelt und die abgetrennte wässrige Phase mit Aethylacetat extrahiert. Die Aethylacetat-Extrakte werden mit Wasser gewaschen, getrocknet und filtriert. Das Filtrat wird zu 14,3 g (100%) rohem Methansulfonsäure-(4S,5S)-4-benzyl-2-oxo-oxazolidin-5-ylmethylester eingeengt, DC ($SiO_2$, Aethylacetat): $R_f$ = 0,4; MS (EI): 286 ($M+H^+$).

Beispiel 10 (6→VII, $R^1$ = o-$NO_2C_6H_4$)

Analog Beispiel 9 erhält man aus 50 g des Alkohols von Beispiel 8 und 80 g o-Nitrobenzolsulfochlorid 88,2 g (93%) o-Nitrobenzolsulfonsäure-(4S,5S)-4-benzyl-2-oxo-oxazolidin-5-ylmethylester, DC ($SiO_2$, Aethylacetat): $R_f$ = 0,38; MS (EI): 393 ($M+H^+$).

Beispiel 11 (6→VII, $R^1$ = p-Tolyl)

Zu einer gerührten Suspension von 5,55 g des Alkohols von Beispiel 8 in 27 ml Aceton und 6,64 g p-Toluolsulfochlorid wird bei 22°C 5,6 ml Triäthylamin gegeben und 6,5 Stunden bei 22°C gerührt. Die Suspension wird mit Wasser versetzt, bei 10°C gerührt, abfiltriert, der Rückstand mit einer Wasser/Aceton-Lösung (3:2) gewaschen und zu 9,09 g (94%) 99%-igem p-Toluolsulfonsäure-(4S,5S)-4-benzyl-2-oxo-oxazolidin-5-ylmethylester, Smp. 148,7-150,3°C, $[\alpha]_D^{20}$ : +3,0° (1% in Aceton) getrocknet.

Beispiel 12 (6→VII, $R^1$ = p-$NO_2C_6H_4$)

Zu einer gerührten Suspension von 12,0 g des Alkohols von Beispiel 8 in 35 ml THF und 7,66 ml N-Methylmorpholin wird bei 20-25°C in Portionen 15,4 g p-Nitrobenzolsulfochlorid gegeben und 4,5 Stunden gerührt. Die Suspension wird erneut mit 0,77 ml N-Methylmorpholin versetzt und 4,5 Stunden bei 22°C gerührt. Das Gemisch wird mit70 ml einer 2%-igen Natriumbicarbonat-Lösung versetzt, 1,5 Stunden gerührt, filtriert, der Rückstand mit Wasser und Aethanol gewaschen und zu 21,4 g (94%) 98,5-igem p-Nitrobenzolsulfonsäure-(4S,5S)-4-benzyl-2-oxo-oxazolidin-5-ylmethylester, Smp. 148-149,5°C, $[\alpha]_D^{20}$ : +10,0° (1% in Aceton) getrocknet.

Beispiel 13 (6→VII, $R^1$ = p-$BrC_6H_4$)

Analog Beispiel 12 erhält man aus 35 g des Alkoholproduktes von Beispiel 8 und 56,1 g p-Brombenzolsulfochlorid 65,5 g (91%) p-Brombenzolsulfonsäure-(4S,5S)-4-benzyl-2-oxo-oxazolidin-5-ylmethylester, Smp. 151,6-153°C.

Beispiel 14 (1→II; $R^3$ = Ac)

Einem Gemisch von 1,76 g NaCN und 0,16 g Benzyltriäthylammoniumchlorid in 30 ml $CH_2Cl_2$ und 55 ml Wasser wird unter Rühren bei 0°C eine Lösung von 5 g 3-Phenyl-2(S)-phthalimidopropan-1-al und 3,66 g Essigsäureanhydrid in 30 ml $CH_2Cl_2$ zugetropft und 7 Stunden bei 0°C weitergerührt. Die wässrige Phase wird mit $CH_2Cl_2$ extrahiert. Die Extrakte werden getrocknet, filtriert und das Filtrat eingeengt. Der Rückstand wird an Kieselgel mit $CH_2Cl_2$:Isopropanol (100:1) chromatographiert. Man erhält 6,12 g (98%) eines 75:25-Gemisches von (2S,3S)- und (2R,3S)-3-(1,3-Dioxo-1,3-dihydroisoindol-2-yl)-2-acetoxy-4-phenylbutyronitril, IR (Film): 2227w (CN), 1773s, 1760s und 1717s (C=O von Imid und Acetat).

Beispiel 15 (1→II; $R^3$ = Benzyl-OCO, H)

A) Eine Lösung von 11,16 g 3-Phenyl-2(S)-phthalimidopropan-1-al und 0,7 g Benzyltriäthylammoniumchlorid in 70 ml $CH_2Cl_2$ wird unter Rühren bei -10°C mit 6,2 ml Chlorameisensäurebenzylester versetzt. Dann wird eine Lösung von 3,10 g NaCN in 50 ml $H_2O$ zugetropft. Nach 0,5 Stunden bei -10°C wird auf 0°C erwärmt. Die $CH_2Cl_2$-Phase wird mit Wasser und mit gesättigter NaCl-Lösung gewaschen. Die wässrige Phase wird mit $CH_2Cl_2$ extrahiert. Die $CH_2Cl_2$-Phasen werden getrocknet, filtriert und das Filtrat eingeengt. Der Rückstand enthält 18,33 g eines 70:30-Gemisches von (2S,3S)- und (2R,3S)-3-(1,3-Dioxo-1,3-dihydroisoindol-2-yl)-2-benzyloxycarbonyloxy-4-phenylbutyronitril, MS (EI): 349 (6, $M^+$-$C_6H_5CH_2$), 288 (6, $M^+$-$C_6H_5CH_2OCOOH$), 91(100, $C_6H_5CH_2$); IR (Film): 2240w (CN), 1763s und 1717s (C=O von Imid und Carbamat).

B) Eine Suspension von 2 g des Benzylcarbonats von A) und 0,15 g Pd/C (10%) in 12 ml Aethanol und 2 ml $CH_2Cl_2$ wird 2 Stunden bei 22°C hydriert, dann filtriert, mit $CH_2Cl_2$ gewaschen und das Filtrat eingeengt. Man erhält 1,35 g (97%) eines 73:27-Gemisches von (2S,3S)- und (2R,3S)-3-(1,3-Dioxo-1,3-dihydroisoindol-2-yl)-2-hydroxy-4-phenylbutyronitril, IR (KBr): 3450m (OH), 2250w (C≡N), 1775m und 1712s (C=O von Imid).

Beispiel 16 (I→II; $R^3$ = H)

Eine Suspension von 5 g 3-Phenyl-2(S)-phthalimidopropan-1-al und 4,43 g $ZnBr_2$ in 50 ml $CH_2Cl_2$ wird unter Rühren bei -15°C mit einer Lösung von 1,95 g Trimethylsilylcyanid in 5 ml $CH_2Cl_2$ versetzt und 5 Stunden bei -15°C gerührt. Der gebildete Silyläther wird durch Zugabe einer Lösung von 5 g Zitronensäure in 50 ml Aethanol bei -10°C gespalten. Das Gemisch wird eingeengt und der Rückstand mit $H_2O$ versetzt und mit $CH_2Cl_2$ extrahiert. Die organischen Extrakte werden getrocknet, filtriert und das Filtrat eingeengt. Der Rückstand enthält 5,45 g (99%) rohes 3-(1,3-Dioxo-1,3-dihydroisoindol-2-yl)-2-hydroxy-4-phenylbutyronitril, als 74:26-Gemisch von (2S,3S) und (2R,3S), IR (KBr): 3437m (OH), 2250w (C≡N), 1775m und 1713s (C=O von Imid).

Beispiel 17 (II, $R^3$ = H, via 1)

A) Eine Suspension von 82,6 g L-Phenylalanin und 74,1 g Phthalsäureanhydrid in 600 ml Toluol wird 8 Stunden unter Argon und Rückfluss erhitzt. Die entstandene Suspension wird auf 22°C abgekühlt und mit 0,5 ml DMF gefolgt von 66,64 g Oxalylchlorid versetzt. Nach 2 Stunden Rühren wird Argon in die Suspension geblasen.

B) Die das 3-Phenyl-2(S)-phthalimidopropionylchlorid enthaltende Lösung wird mit 500 ml Toluol verdünnt und mit 72,11 g 1,2-Butylenoxid versetzt. Der Lösung weden 23,5 g Pd/C (5%) und 100 ml Toluol zugesetzt. Die Suspension wird 17 Stunden unter Rühren hydriert, dann filtriert und der Rückstand mit 200 ml Toluol gewaschen.

C) Der das 3-Phenyl-2(S)-phthalimidopropan-1-al enthaltenden Lösung wird bei 22°C unter Rühren eine Lösung von 95,05 g Natriumpyrosulfit in 1 l Wasser zugesetzt. Nach 4,5 Stunden Rühren wird die das Additionsprodukt von Bisulfit und des obigen Aldehyds enthaltende wässrige Schicht mit Toluol gewaschen. Die Toluolschichten werden mit Wasser extrahiert. Den Wasserschichten werden 1200 ml $CH_2Cl_2$ zugesetzt und das Gemisch wird unter Rühren bei 22°C mit einer Lösung von 41,66 g NaCN in 330 ml Wasser versetzt. Nach 1,2 Stunden Rühren wird Wasser zugesetzt. Die abgetrennte wässrige Schicht wird mit $CH_2Cl_2$ extrahiert. Die organischen Schichten werden getrocknet, filtriert und der Rückstand wird mit $CH_2Cl_2$ gewaschen. Die Filtrate werden eingedampft und der Rückstand in 200 ml $CH_2Cl_2$ gelöst. Der Lösung wird unter Rühren bei 30°C 600 ml Hexan und dann bei 0°C nochmals 600 ml Hexan zugesetzt. Die Suspension wird filtriert, der Rückstand mit Hexan gewaschen und dann getrocknet. Man erhält 114,02 g (74%) eines 74,7:23,5:1,4:0,4-Gemisches der Isomeren (2S,3S):(2R,3S):(2R,3R):(2S,3R) von 3-(1,3-Dioxo-1,3-dihydroisoindol-2-yl)-2-hydroxy-4-phenylbutyronitril, Smp. 127,2-130,5°C, $[\alpha]_D^{20}$ : -146,6° (1% in $CH_2Cl_2$).

Beispiel 18 (I→II, $R^3$ = H)

Der das Produkt von Beispiel 17B), das 3-Phenyl-2(S)-phthalimidopropan-1-al, enthaltenden Lösung wird bei 22°C unter Rühren eine Lösung von 47,5 g Natriumpyrosulfit in 500 ml Wasser zugesetzt. Nach 7,5 Stunden Rühren wird die das Additionsprodukt von Pyrosulfit und des obigen Aldehyds enthaltende wässrige Schicht mit Toluol gewaschen. Die Toluolschichten werden mit Wasser extrahiert. Den Wasserschichten wird unter Rühren bei 25°C eine Lösung von 24,2 g NaCN in 200 ml Wasser zugesetzt. Die Suspension wird nach 1 Stunde Rühren filtriert, der Rückstand mit Wasser neutral gewaschen. Nach Trocknen erhält man 112,03 g (73%) eines 67,2:32,8-Gemisches der Isomeren (2S,3S):(2R,3S) von 3-(1,3-Dioxo-1,3-dihydroisoindol-2-yl)-2-hydroxy-4-phenylbutyronitril, Smp. 131-133°C, $[\alpha]_D^{20}$ : -150,2° (1% in $CH_2Cl_2$).

Beispiel 19 (II→III, $R^2$ = $CH_3$, $R^3$ = $R^4$ = H)

A) Einer Lösung von 400 g HCl in 980 ml Methanol wird bei 0°C eine Lösung von 100 g des 3-(1,3-Dioxo-1,3-dihydroisoindol-2-yl)-2-hydroxy-4-phenylbutyronitrils (Beispiel 17C) in 450 ml $CH_2Cl_2$ zugesetzt. Nach 18 Stunden Rühren bei 0°C wird die Suspension filtriert. Der den entstandenen Iminoäther•HCl enthaltende Rückstand wird einem Gemisch von je 600 ml Aethylacetat und Wasser zugesetzt. Nach dem Auflösen des Festkörpers wird die wässrige Schicht mit Aethylacetat extrahiert. Die organischen Schichten werden mit gesättigter $NaHCO_3$-Lösung und mit Wasser gewaschen und dann getrocknet. Die Suspension wird filtriert und das Filtrat zu 77,15 g (70%) eines 93:7-Gemisches der (2S,3S):(2R,3S)-Isomeren des 3-(1,3-Dioxo-1,3-dihydroisoindol-2-yl)-2-hydroxy-4-phenylbuttersäuremethylesters, $[\alpha]_D^{20}$ : -121,5° (1% in Aethylacetat) eingedampft.

B) Zu einer Lösung von 200 g des 3-(1,3-Dioxo-1,3-dihydroisoindol-2-yl)-2-hydroxy-4-phenylbutyronitrils (Beispiel 17C) in 1200 ml Toluol und 106 ml Methanol werden bei 0°C 120 g HCl eingeleitet und 3 Stunden bei 0°C gerührt. Die Suspension wird mit 1200 ml Wasser und 400 ml Methanol versetzt und 2 Stunden bei 22°C gerührt. Die wässrige Schicht wird mit Toluol extrahiert. Die organischen Schichten werden mit Wasser gewaschen, getrocknet, filtriert und das Filtrat wird zu 221 g (100%) eines 75:25-Gemisches der (2S,3S):(2R,3S)-Isomeren des 3-(1,3-Dioxo-1,3-dihydroisoindol-2-yl)-2-hydroxy-4-phenylbuttersäuremethylester, $[\alpha]_D^{20}$ : -133,1° (1% in Aethylacetat) eingedampft.

Beispiel 20 (II→III, $R^2$ = $C_2H_5$, $R^3$ = $R^4$ = H)

150 ml mit HCl gesättigtem Aethanol wird bei 0°C mit einer Lösung von 30 g des 3-(1,3-Dioxo-1,3-dihydroisoindol-2-yl)-2-hydroxy-4-phenylbutyronitrils (aus Beispiel 17C) in 150 ml $CH_2Cl_2$ versetzt. Nach 4 Stunden bei 0°C wird die Suspension mit 150 ml $CH_2Cl_2$ verdünnt, abfiltriert und der Rückstand mit $CH_2Cl_2$ gewaschen. Zur Hydrolyse des gebildeten Iminoäther•HCl wird der Rückstand in 250 ml Toluol, 250 ml Wasser und 12 ml Aethanol aufgenommen. Nach 4 Stunden Rühren wird die wässrige Phase mit Toluol extrahiert und die Toluolphasen mit Wasser gewaschen. Die Toluolphasen werden getrocknet, filtriert und das Filtrat eingeengt. Der Rückstand enthält 19,54 g (57%) eines 95:5-Gemisches von (2S,3S)- und (2R,3S)-3-(1,3-Dioxo-1,3-dihydroisoindol-2-yl)-2-hydroxy-4-phenylbuttersäureäthylester, Smp. 83,1-84,3°C, $[\alpha]_D^{20}$ = -143,1° (1% in Aethylacetat).

Beispiel 21 (II→III, $R^2$ = i-Pr, $R^3$ = $R^4$ = H)

A) 150 ml mit HCl gesättigtes Isopropanol werden unter Rühren bei 0°C mit einer Lösung von 30 g des 3-(1,3-Dioxo-1,3-dihydroisoindol-2-yl)-2-hydroxy-4-phenylbutyronitrils aus Beispiel 17C) in 150 ml $CH_2Cl_2$ versetzt. Nach 9 Stunden bei 0°C wird die Lösung mit 300 ml $H_2O$ versetzt und 4 Stunden bei 22°C gerührt. Die Phasen werden getrennt und die wässrige Phase mit $CH_2Cl_2$ extrahiert. Die organischen Extrakte werden mit Wasser gewaschen, getrocknet, filtriert und das Filtrat eingeengt. Der Rückstand enthält 29,73 g (83%) eines 70:30-Gemisches der (2S,3S)- und (2R,3S)-Isomeren des 3-(1,3-Dioxo-1,3-dihydroisoindol-2-yl)-2-hydroxy-4-phenylbuttersäureisopropylester, $[\alpha]_D^{20}$ = -126,8° (1% in Aethylacetat); MS (EI): 368 (1, $M+H^+$), 250 [100, M-CH(OH)COOCH$(CH_3)_2$].

B) Das Isomerengemisch von A) wird in 180 ml t-Butylmethyläther gelöst, mit 60 ml Hexan versetzt, bei 22°C und dann bei 0°C und -10°C gerührt und abfiltriert. Nach Trocknen erhält man 13,13 g (36,5%) (2S,3S)-3-(1,3-Dioxo-1,3-dihydroisoindol-2-yl)-2-hydroxy-4-phenylbuttersäureisopropylester, IR (Film): 3463m (OH), 1773m, 1730s und 1705s (C=O von Imid und Ester).

Beispiel 22 (II→III, $R^2$ = $CH_3$, $R^3$ = Ac, $R^4$ = H)

Eine Lösung von 3,39 g eines 75:25-Gemisches von (2S,3S)- und (2R,3S)-3-(1,3-Dioxo-1,3-dihydroisoindol-2-yl)-2-acetoxy-4-phenylbutyronitril (Beispiel 14) in 25 ml Methanol wird bei 0°C mit HCl gesättigt und 21 Stunden bei 0°C gerührt. Die Lösung wird mit 100 ml Wasser versetzt und mit Aethylacetat extrahiert. Die Extrakte werden getrocknet, filtriert und das Filtrat wird eingeengt. Der Rückstand enthält 3,3 g (100%) eines 75:25-Gemisches der (2S,3S):(2R,3S)-Isomeren des 3-(1,3-Dioxo-1,3-dihydroisoindol-2-yl)-2-hydroxy-4-phenylbuttersäuremethylesters, IR (Film): 3362m (OH), 1775m, 1745s und 1712s (C=O von Imid und Ester).

Beispiel 23 (III→IV, $R^2$ = $CH_3$)

A) Eine Lösung von 20 g eines 93:7-Gemisches der (2S,3S):(2R,3S)-Isomeren des 3-(1,3-Dioxo-1,3-dihydroisoindol-2-yl)-2-hydroxy-4-phenylbuttersäuremethylesters (Beispiel 19A) in 20 ml Methanol wird unter Rühren bei 0°C mit einer Lösung von 11,75 ml einer 18,7%igen Lösung von Methylamin in Methanol behandelt und 4 Stunden bei 0°C gerührt.

Der Lösung werden 44 ml einer 20%igen Lösung von HCl in Methanol bei 0°C zugefügt. Nach 3 Stunden Rühren bei 22°C wird die Suspension filtriert, der Rückstand mit Methanol gewaschen und das Filtrat eingeengt. Bei 0°C wird der pH des Rückstandes mit verdünnter Ammoniaklösung auf 4 gestellt. Die wässrige Schicht wird mit Aethylacetat gewaschen, die organische Schicht mit Wasser extrahiert und der pH der vereinigten Wasserschichten bei 22°C mit Ammoniaklösung auf 9,3 gestellt. Die wässrige Schicht wird mehrmals mit Aethylacetat extrahiert, die organischen Schichten getrocknet, filtriert und das Filtrat auf 50 g eingeengt. Die zurückbleibende Lösung wird mit 3,4 ml Essigsäure behandelt und bei 0°C gerührt. Die Suspension wird filtriert und der Rückstand mit Aethylacetat gewaschen. Nach Trocknen erhält man 13,83 g (87%) eines 98:2-Gemisches der (2S,3S)- und (2R,3S)-Isomeren des 3-Amino-2-hydroxy-4-phenylbuttersäuremethylester-acetats, Smp. 113-114,5°C, $[\alpha]_D^{20}$ : +15,3° (1% in Methanol).

B) Analog Beispiel 23A) erhält man durch Umsetzung von 54,6 g eines 75:25-Gemisches der (2S,3S):(2R,3S)-Isomeren des 3-(1,3-Dioxo-1,3-dihydroisoindol)-2-yl)-2-hydroxy-4-phenylbuttersäuremethylesters (Beispiel 19B) 28,4 g (66%) eines 92:8-Gemisches der (2S,3S)- und (2R,3S)-Isomeren des 3-Amino-2-hydroxy-4-phenylbuttersäuremethylester-acetats, Smp.

109-110 °C, $[\alpha]_D^{20}$ : +13,6 ° (1% in Methanol).

Beispiel 24 (III→IV, R2 = $C_2H_5$)

Analog Beispiel 23 erhält man durch Umsetzung von einer Lösung von 10,42 g eines 95:5-Gemischs der (2S,3S)- und (2R,3S)-Isomeren des 3-(1,3-Dioxo-1,3-dihydroisoindol-2-yl)-2-hydroxy-4-phenylbuttersäureäthylesters (Beispiel 20) in 10 ml Aethanol mit 5,9 ml einer 33%igen Lösung von Methylamin in Aethanol 5,39 g (82%) (2S,3S)-3-Amino-2-hydroxy-4-phenylbuttersäureäthylester, DC ($SiO_2$, $CH_2Cl_2$/Methanol 10:1, $UV_{254}$): $R_f$ = 0,34.

**Patentansprüche**

1. Verfahren zur Herstellung des 2-[3(S)-Amino-2-(R)-hydroxy-4-phenylbutyl]-N-tert.butyl-decahydro-(4aS,8aS)-isochinolin-3(S)-carboxamids der Formel

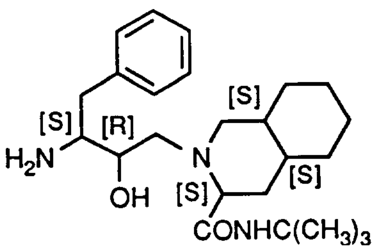

10

dadurch gekennzeichnet, dass man

1.a) einen Sulfonsäureester der Formel

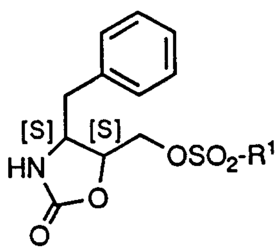

VII

worin $R^1$ nieder-Alkyl oder gegebenenfalls durch bis zu 2 Halogenatome oder nieder-Alkyl- oder Nitrogruppen substituiertes Phenyl ist,

in Gegenwart von einer Base mit dem N-tert.Butyl-decahydro-(4aS,8aS)-isochinolin-3(S)-carboxamid der Formel

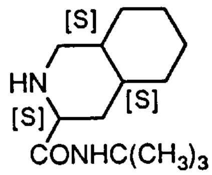

8

umsetzt, das entstandene Oxazolidinon der Formel

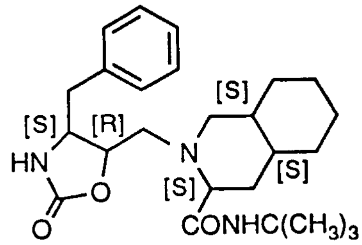

9

mit einer starken Säure in ein Salz des Oxazolidinons der Formel 9 überführt, dieses Salz mit einer Base versetzt und

1.b) das erhaltene Oxazolidinon der Formel 9 mit einer Base zum Aminoalkohol der obigen Formel 10 aufspaltet.

2. Verfahren zur Herstellung der Sulfonsäureester der Formel VII nach Anspruch 1, dadurch gekennzeichnet, dass man

2.a) einen α-Hydroxy-β-aminosäureester der Formel

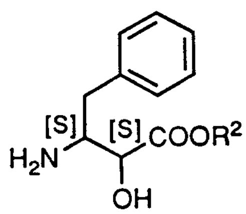

IV

worin $R^2$ nieder-Alkyl ist,

oder ein Salz davon mittels eines Carbonylierungsmittels cyclisiert,

2.b) im so erhaltenen 2-Oxo-oxazolidin-5-carbonsäureester der Formel

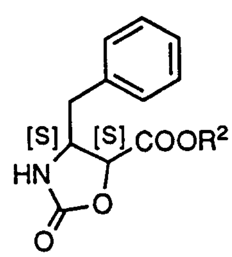

V

die Carbo-niederalkoxygruppe zur Hydroxymethylgruppe reduziert und

2.c) den erhaltenen (4S,5S)-4-Benzyl-5-hydroxymethyl-oxazolidin-2-on der Formel

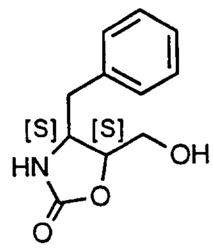

6

in Gegenwart einer Base mit einem Sulfonsäurechlorid $R^1$-$SO_2Cl$ in einen Sulfonsäureester VII umwandelt.

**3.** Verfahren zur Herstellung der α-Hydroxy-β-aminosäureester der Formel IV nach Anspruch 2, dadurch gekennzeichnet, dass man

3.a) das 3-Phenyl-2(S)-phthalimidopropan-1-al der Formel

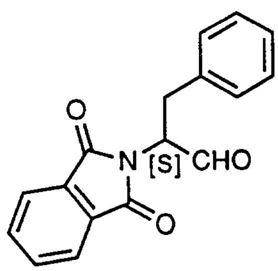

1

in das 1-Cyano-3-phenyl-2(S)-phthalimidopropan-1-olderivat der Formel

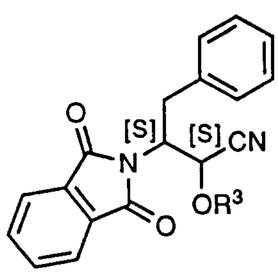

II

worin $R^3$ H, nieder-Alkanoyl, Benzyloxycarbonyl oder Triniederalkylsilyl ist, umwandelt,

3.b) eine im Nitril der Formel II enthaltene Triniederalkylsilylgruppe abspaltet,

3.c) das erhaltene Nitril der Formel II, worin $R^3$ H, nieder-Alkanoyl oder Benzyloxycarbonyl ist, in Gegenwart eines nieder-Alkanols $R^2$-OH mit einer starken Säure in ein Salz des Iminoäthers der Formel

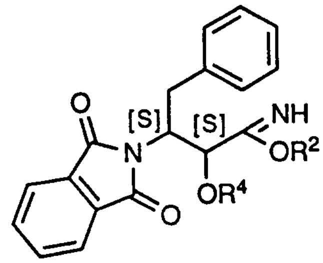

II'

worin $R^4$ H, nieder-Alkanoyl oder Benzyloxycarbonyl und $R^2$ nieder-Alkyl ist, umwandelt,

3.d) das Salz des Iminoäthers der Formel II' gegebenenfalls isoliert, dann hydrolysiert und

3.e) den erhaltenen α-Hydroxysäureester der Formel

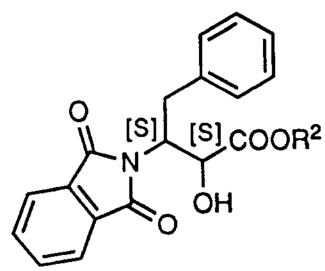

III

zunächst mit einer Base und dann einer starken Säure in den α-Hydroxy-β-aminosäureester der Formel IV umwandelt.

**4.** Verfahren nach Anspruch 1, 2 oder 3, worin $R^1$ Methyl, p-Tolyl, p-Bromphenyl oder o-Nitrophenyl, $R^2$ Aethyl oder Isopropyl, $R^3$ und $R^4$ H, Acetyl oder Benzyloxycarbonyl oder $R^3$ Trimethylsilyl, und insbesondere worin $R^1$ p-Nitrophenyl, $R^2$ Methyl und $R^3$ und $R^4$ H sind.

**5.** Die Salze der Verbindung der Formel 9 mit starken Säuren und die Verbindungen der Formeln II bis V, 6 und VII nach den Ansprüchen 1, 2 und 3.

**6.** Die Salze und Verbindungen nach Anspruch 5 aus der Gruppe der folgenden:

das (3S,4aS,8aS)-2-[(4S,5R)-4-Benzyl-2-oxo-oxazolidin-5-ylmethyl]-N-tert.butyl-decahydro-isochinolin-3-carboxamid-p-tolylsulfonat,

(3S,4aS,8aS)-2-[(4S,5R)-4-Benzyl-2-oxo-oxazolidin-5-ylmethyl]-N-tert.butyl-decahydro-isochinolin-3-carboxamid-p-bromphenylsulfonat und insbesondere

(3S,4aS,8aS)-2-[(4S,5R)-4-Benzyl-2-oxo-oxazolidin-5-ylmethyl]-N-tert.butyl-decahydro-isochinolin-3-carboxamid-p-nitrobenzolsulfonat;

das Methansulfonsäure-(4S,5S)-4-benzyl-2-oxo-oxazolidin-5-ylmethylester,

o-Nitrobenzolsulfonsäure-(4S,5S)-4-benzyl-2-oxo-oxazolidin-5-ylmethylester,

p-Toluolsulfonsäure-(4S,5S)-4-benzyl-2-oxo-oxazolidin-5-ylmethylester,

p-Brombenzolsulfonsäure-(4S,5S)-4-benzyl-2-oxo-oxazolidin-5-ylmethylester und insbesondere

p-Nitrobenzolsulfonsäure-(4S,5S)-4-benzyl-2-oxo-oxazolidin-5-ylmethylester;

das (4S,5S)-4-Benzyl-5-hydroxymethyl-oxazolidin-2-on;

der (4S,5S)-4-Benzyl-2-oxo-oxazolidin-5-carbonsäuremethylester;

der (2S,3S)-3-Amino-2-hydroxy-4-phenylbuttersäureäthylester und inbesondere

(2S,3S)-3-Amino-2-hydroxy-4-phenylbuttersäuremethylester;

der (2S,3S)-3-(1,3-Dioxo-1,3-dihydroisoindol-2-yl)-2-hydroxy-4-phenylbuttersäureäthylester,

(2S,3S)-3-(1,3-Dioxo-1,3-dihydroisoindol-2-yl)-2-hydroxy-4-phenylbuttersäureisopropylester und insbesondere

(2S,3S)-3-(1,3-Dioxo-1,3-dihydroisoindol-2-yl)-2-hydroxy-4-phenylbuttersäuremethylester;

das (2S,3S)-3-(1,3-Dioxo-1,3-dihydroisoindol-2-yl)-2-acetoxy-4-phenylbutyronitril,

(2S,3S)-3-(1,3-Dioxo-1,3,dihydroisoindol-2-yl)-2-benzyloxycarbonyloxy-4-phenylbutyronitril und insbesondere

(2S,3S)-3-(1,3-Dioxo-1,3-dihydroisoindol-2-yl)-2-hydroxy-4-phenylbutyronitril.